# EUROPEAN PATENT SPECIFICATION

(11) **EP 2 621 268 B1**
(45) Date of publication and mention of the grant of the patent: **05.04.2017**
(21) Application number: 11829785.2
(22) Date of filing: 26.09.2011
(51) Int. Cl.: A01K 67/00, C12N 15/00, A01K 67/027, C12N 15/85, G01N 33/50

(54) **MODEL FOR MUSCLE ATROPHY**
MODELL FÜR MUSKELATROPHIE
MODÈLE D'ATROPHIE MUSCULAIRE

(30) Priority: 30.09.2010 US 388459 P
(43) Date of publication of application: 07.08.2013
(73) Proprietor: Rigel Pharmaceuticals, Inc., South San Francisco, CA 94080 (US)
(72) Inventor: KINSELLA, Todd M., Redwood City, CA 94061 (US); PAYAN, Donald G., Hillsborough, CA 94010 (US)
(74) Representative: Brasnett, Adrian Hugh
(86) International application number: PCT/US2011/053298
(87) International publication number: WO 2012/044585

(56) References cited:
- JP-A- 2010 184 879
- DATABASE WPI Week 201057 Thomson Scientific, London, GB; AN 2010-K57266 XP002720244, & JP 2010 184879 A (UNIV TOKYO) 26 August 2010 (2010-08-26)
- WADDELL DAVID S ET AL: "The glucocorticoid receptor and FOXO1 synergistically activate the skeletal muscle atrophy-associated MuRF1 gene", AMERICAN JOURNAL OF PHYSIOLOGY - ENDOCRINOLOGY AND METABOLISM, vol. 295, no. 4, October 2008 (2008-10), pages E785-E797, XP002720245, ISSN: 0193-1849
- CAI DONGSHENG ET AL: "IKKbeta/NF-kappaB activation causes severe muscle wasting in mice", CELL, vol. 119, no. 2, 15 October 2004 (2004-10-15), pages 285-298, XP002445877, CELL PRESS, US ISSN: 0092-8674, DOI: 10.1016/J.CELL.2004.09.027
- VIVIANA MORESI ET AL: "Myogenin and Class II HDACs Control Neurogenic Muscle Atrophy by Inducing E3 Ubiquitin Ligases", CELL, vol. 143, no. 1, 1 October 2010 (2010-10-01), pages 35-45, XP055038436, ISSN: 0092-8674, DOI: 10.1016/j.cell.2010.09.004
- HIRNER STEPHANIE ET AL: "MuRF1-dependent regulation of systemic carbohydrate metabolism as revealed from transgenic mouse studies", JOURNAL OF MOLECULAR BIOLOGY, vol. 379, no. 4, June 2008 (2008-06), pages 666-677, XP002720246, ISSN: 0022-2836
- WILLIS MONTE S ET AL: "Cardiac Muscle Ring Finger-1 Increases Susceptibility to Heart Failure In Vivo", CIRCULATION RESEARCH, vol. 105, no. 1, July 2009 (2009-07), pages 80-88+23PP, XP002720247, ISSN: 0009-7330
- BODINE ET AL.: 'Identification of ubiquitin ligases required for skeletal muscle atrophy.' SCIENCE vol. 294, 2001, pages 1704 - 1708, XP002386330
- NISHIJO ET AL.: 'Biomarker system for studying muscle, stem cells, and cancer in vivo.' FASEB J vol. 23, 2009, pages 2681 - 2690, XP008162962

## Description

### BACKGROUND

A general loss of muscle mass or atrophy is a characteristic response to fasting, as well as many diseases, including advanced cancer, renal failure, sepsis, cachexia, arthritis, osteoporosis, and diabetes. Atrophy of muscles also results from their disuse or denervation, e.g., immobilization, muscle unloading, spinal cord injury etc. Atrophy contributes substantially to many common health problems, including but not limited to HIV, chronic heart failure, chronic kidney disease, liver cirrhosis, burn injuries, osteoporosis, arthritis etc. Regardless of the cause of muscle atrophy, skeletal muscle atrophy is characterized by a decrease in protein content, fiber diameter, force production, and fatigue resistance.

Loss of muscle mass associated with progression of atrophy has been studied in animals subjected to denervation, immobilization, starvation, and animals implanted with cancer cells capable of inducing muscle wasting. Alternatively, atrophy can be induced in animals subjected to glucocorticoid administration. In these animals, the degree of muscle wasting can be assessed by employing a variety of measurements that record changes in muscle weights or fiber cross sectional area, and by performing kinetic experiments using a large number of animals etc. Detecting a significant change in muscle mass or in kinetics often requires a long waiting period. Measurement of muscle weight and fiber cross sectional area require cumbersome surgical procedures, cross-sectional area measurements and often the animal is sacrificed. Thus, such procedures usually require a large number of animals and precludes being able to follow a set of muscles, temporally, in the same animal.

Certain aspects of this disclosure relate to cell lines and animal models for monitoring initiation and/or progression of muscle atrophy.

### SUMMARY

A non-human animal model for muscle cell atrophy is provided as defined in claim 1. The animal comprises muscle cells comprising a nuclear genome comprising a biologically active atrogen gene in operable linkage with a reporter construct comprising: i. a first coding sequence for fluorescent or luminescent detectable protein; and ii. a second coding sequence for a secreted reporter enzyme. The atrogen gene, the detectable protein and the secreted reporter enzyme are co-expressed in that initiation of muscle cell atrophy results in production of the secreted reporter enzyme and the detectable protein by the cell. Screening assays that employ the animal are also provided.

In one embodiment, the biologically active atrogen gene is endogenous to the cell, whereas in other embodiments, the biologically active atrogen gene is not endogenous to said cell. For example, the cell may further comprise an endogenous atrogen gene, and said biological active atrogen gene is comprised in a recombinant construct that is present at a different locus to the endogenous atrogen gene.

In particular embodiments, induction of the atrogen promoter by muscle cell atrophy results in secretion of the secreted reporter enzyme into the bloodstream of the animal, thereby allowing the induction of the atrogen gene to be monitored in the blood of the animal. The optically-detectable protein, e.g., luciferase, may be used to detect where in the animal muscle cell atrophy occurs.

In particular embodiments, the reporter construct may be present in the 3' UTR of the atrogen gene. In some of these embodiments, the reporter construct may encode a translational shunt sequence that is present between the first coding sequence and the second coding sequence, thereby allowing co-expression of the two reporter proteins. In addition, the reporter construct may also encode a translational shunt sequence that is present between the final exon of the atrogen gene the first coding sequence, thereby allowing co-expression of the atrogen gene and the reporter proteins. In other embodiments, the first and second coding sequences may be operably linked to the atrogen gene via an internal ribosome entry site (IRES). The reporter construct may, in certain cases, encode a translational shunt sequence that is present between the first coding sequence and the second coding sequence.

In particular embodiments, the IRES may be a hepatitis C virus (HCV) virus, and, independently, the translational shunt sequence may be the sequence of the FMDV 2A translational shunt.

In the invention the optically detectable protein is a fluorescent protein or luciferase, for example, and the secreted reporter enzyme may be a secreted akaline phosphatase, for example.

The animal, which in certain embodiments may be a rodent such as a rat, may be homozygous or heterozygous for the atrogen gene.

In particular cases, the cell of the animal may be made by inserting the reporter construct into said 3'UTR of an atrogen gene that is endogenous to said cell. In other cases, the cell may be made by inserting a recombinant nucleic acid comprising said atrogen gene and the reporter construct into said genome at a different locus to an endogenous version of the atrogen gene.

A muscle cell comprising a nuclear genome comprising a biologically active atrogen gene in operable linkage with a reporter construct is also provided as defined in the claims. This cell comprises a first coding sequence for a fluorescent or luminescent detectable protein, and a second coding sequence for a secreted reporter enzyme; wherein expression of the atrogen gene is induced upon initiation of muscle cell atrophy, thereby resulting in production of said secreted reporter enzyme and said detectable protein by said cell.

Also provided is a method of screening. In particular embodiments, this method may comprise: a) contacting the above-described non-human animal with a candidate agent under conditions that induce muscle cell atrophy; and b) determining if the candidate agent alters production of the secreted reporter enzyme and said detectable protein by said animal. In particular embodiments, the method may further comprise: a) administering the candidate agent to an animal under conditions that induce muscle cell atrophy; and b) determining if the candidate agent modulates muscle cell atrophy.

The conditions that induce muscle cell atrophy may comprise contacting the cell with a muscle cell atrophy inducing agent, e.g., an agent that comprises a glucocorticoid.

If an animal is employed in the method, the method may further comprise measuring the activity of the secreted reporter enzyme in the blood of the animal and imaging said animal to detect expression of the optically detectable protein.

In certain embodiments, the animal model may comprise a recombinant cell comprising an atrogen promoter operably linked to a first coding sequence for a secreted reported enzyme and a second coding sequence for an optically detectable protein, where induction of the promoter results in production of the secreted reporter enzyme and the first optically detectable protein by the cell. The construct may be integrated randomly or site-specifically in the genome of the cell. In certain embodiments, the nuclear genome of the cell may comprise: a) an atrogen gene comprising: an atrogen promoter, a coding sequence for an atrogen protein and a 3'UTR; and b) a reporter construct comprising: i) an IRES, ii) a first coding sequence for a secreted reporter enzyme, and iii) a second coding sequence for a first optically detectable protein; where the IRES is operably linked to the first and second coding sequences, where the reporter construct is present in the 3'UTR, and where the atrogen promoter is induced upon initiation of muscle cell atrophy, thereby resulting in production of the secreted reporter enzyme and the first optically detectable protein by the cell. Isolated cells comprising a nuclear genome comprising the reporter construct, e.g., present in 3'UTR of an atrogen gene as described above, and methods for using the animal model and cells are provided.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.
Fig. 1A schematically illustrates an embodiment of a reporter construct. Fig. 1B schematically illustrates another embodiment of a reporter construct.
Fig. 2 shows a plasmid containing a reporter construct.
Fig. 3 shows the sequence for the junction sequences 2A.1, 2A.2, and 2A.3 with flexible linkers (Top panel: SEQ ID NO: 1 and SEQ ID NO: 2; middle panel SEQ ID NO: 3 and SEQ ID NO: 4; and bottom panel: SEQ ID NO: 5 and SEQ ID NO:6, respectively).
Fig. 4 schematically illustrates a reporter construct and its location in the genomic DNA of a transgenic mouse. Detection of reporter proteins in the mouse by *in vivo* imaging is also shown
Fig. 5 schematically illustrates a reporter construct and its location in the genomic DNA of a transgenic mouse. The lower panel shows another construct integrated into the genome of this mouse. Detection of reporter proteins in the mouse by *in vivo* imaging is also shown.
Fig. 6 depicts 3D-tomography *in vivo* imaging of whole animal to detect muscles expressing a fluorescence/bioluminescence reporter protein.
Fig. 7 illustrates a configuration in which a reporter construct is inserted into the 3' UTR of an endogenous MuRF1 gene. In this embodiment, translational shunt sequences are employed to provide co-expression of MuRF1, luciferase and SEAP.
Fig. 8 illustrates a configuration in which a reporter construct is inserted into the 3' UTR of an endogenous Atrogin-1 gene. In this embodiment, translational shunt sequences are employed to provide co-expression of Atrogin-1, luciferase and SEAP.
Fig. 9 illustrates a configuration in which a reporter construct is inserted into the 3' UTR of a MuRF-1 gene that is present in a BAC. In this embodiment, an IRES and a translational shunt sequence are employed to provide co-expression of MuRF1, luciferase and SEAP.

### DEFINITIONS

The terms "determining", "measuring", "evaluating", "assessing" and "assaying" are used interchangeably herein to refer to any form of measurement, and include determining if an element is present or not. These terms include both quantitative and/or qualitative determinations. Assessing may be relative or absolute. "Determining the presence of" includes determining the amount of something present, as well as determining whether it is present or absent.

The term "contacting" means to bring or put together. As such, a first item is contacted with a second item when the two items are brought or put together, e.g., by touching them to each other or combining them in the same solution.

The phrase "optical signal" refers to light signal that can be detected by a photodetector, e.g., a light microscope, a spectrophotometer, a fluorescent microscope, a fluorescent sample reader, or a fluorescence activated cell sorter, 3D tomographer, a camera, etc.

The term "optically detectable protein" refers to a protein whose expression can be detected by the presence of an optical signal produced by the protein. An optical signal is produced by a protein, for example, when the protein is capable of being excited by a particular wavelength of light and emits another wavelength of light which is detectable. An optical signal is produced by a protein, for example, when the protein catalyzes a reaction which results in a light signal. Fluorescent proteins, luminescent proteins, etc., are examples of optically detectable proteins.

The term "gene" refers to a nucleic acid sequence comprised of a promoter region, a coding sequence, and a 3'UTR.

The terms "protein" and "polypeptide" are used interchangeably herein.

A "signal sequence" is a sequence of amino acids present at the N-terminal portion of a protein which facilitates the secretion of the mature form of the protein from the cell. The definition of a signal sequence is a functional one. The mature form of the extracellular protein lacks the signal sequence, which is cleaved off during the secretion process.

The term "nucleic acid" encompasses DNA, RNA, single stranded or double stranded and chemical modifications thereof. The terms "nucleic acid" and "polynucleotide" are used interchangeably herein.

A "non-human" animal refers to any mammal of a species that is not human.

The terms "rodent" and 'rodents' refer to all members of the phylogenetic order Rodentia including any and all progeny of all future generations derived therefrom.

The term "murine" refers to any and all members of the family Muridae, including rats and mice.

The term "progeny" or "off-spring" refers to any and all future generations derived and descending from a particular mammal. Thus, progeny of any successive generation are included herein such that the progeny, the F1, F2, F3, generations and so on are included in this definition.

The phrase "transgenic animal" refers to an animal comprising cells containing foreign nucleic acid (i.e., recombinant nucleic acid that is not native to the animal). The foreign nucleic acid may be present in all cells of the animal or in some but not all cells of the animal. The foreign nucleic acid molecule is called a "transgene" and may contain one or many genes, cDNA, etc. By inserting a transgene into a fertilized oocyte or cells from the early embryo, the resulting transgenic animal may be fully transgenic and able to transmit the foreign nucleic acid stably in its germline. Alternatively, a foreign nucleic acid may be introduced by transferring, e.g., implanting, a recombinant cell or tissue containing the same into an animal to produce a partially transgenic animal.

The phrase "site specific recombinases" refers to enzymes that are present in some viruses and bacteria and have been characterized to have both endonuclease and ligase properties. Site specific recombinases catalyze at least the following four events (a) deletion of a DNA fragment flanked by "compatible site-specific recombinase targeting sites" (SSRTS) in the same orientation (e.g. head-to-tail or tail-to-head); (b) inversion of a DNA fragment flanked by compatible SSRTS in opposite orientation (e.g. head-to-head or tail-to-tail); (c) integration of a cyclic DNA fragment containing a SSRTS into a compatible SSRTS; and (d) chromosomal translocation between compatible SSRTS located on different chromosomes. To perform those reactions, the site-specific recombinase has typically at least the following four activities: (1) recognition of one or two specific DNA sequences; (2) cleavage of said DNA sequence or sequences; (3) DNA topoisomerase activity involved in strand exchange; and (4) DNA ligase activity to reseal the cleaved strands of DNA (Sauer, B., Cur. Opin. Biotech. 5: 521-527, 1994). The term "recombinase" or "site-specific recombinase" refers to enzyme(s) that carry out site specific recombination (SSR) to alter the DNA structure. This definition includes transposases, lambda integration/excision enzymes, and site-specific recombinases. Well-known examples of recombinases can be found in Cre-lox, FLP/FRT, R/RS, Gin/gix, a pSR1 system, a cer system, and a fim system (e.g. N. L. Craig, Annu. Rev. Genet. 22:17, 1988); Odell et al., Homologous Recomb. Gene Silencing Plants, 1994, pp. 219-270, Paszkowski, Jerzy, ed. Kluwer: Dordrecht, Germany). Additionally, SSR systems have been identified in microorganisms such as phage, bacterium (e.g., *E. coli*), yeast and the like.

The phrase "site-specific recombinase targeting site" or "recombinase site" or "site-specific sequence" means a DNA sequence that a compatible site-specific recombinase will recognize and bind to. It will be appreciated that this may be a wild type or mutant recombinase site, as long as functionality is maintained and the recombinase enzyme may still recognize the site, bind to the DNA sequence, and catalyze the recombination between two adjacent recombinase sites.

The term "floxed" refers to the flanking of a genetic element, or a portion thereof, with tandem site-specific sequences. The site-specific sequences may be oriented in the same orientation, i.e., directly repeated (such that the DNA element is removed upon SSR), or in opposite orientations from one another (such that the DNA element is inverted upon SSR). The floxed element may be a single promoter, a transcriptional "STOP" element, a promoter operably linked to a target sequence, or a combination of these and other genetic elements.

The term "introns" refers to sequences of DNA found in the middle of many gene sequences in most eukaryotes. These intron sequences are transcribed, but removed from within the pre-mRNA transcript before the mRNA is translated into a protein. This process of intron removal occurs by self-splicing of the sequences (exons) on either side of the intron.

The term "operably-linked" refers to the association of nucleic acid sequences on a single nucleic acid fragment so that the function of one is affected by the other. For example, a promoter is operably-linked with a coding sequence when it is capable of affecting the expression of that coding sequence (i.e., the coding sequence is under the transcriptional control of the promoter). Similarly, when an IRES is operably-linked to a coding sequence, the IRES provides for translation of the mRNA transcribed from that coding sequence. Another example of operably linked sequence is the when a sequence is present in the 3'UTR of a gene. This sequence is operably linked to the promoter region of that gene. Two elements that are operably linked includes two elements that are in tandem as well as one element inserted into the other. "Unlinked" means that the associated genetic elements are not closely associated with one another and the function of one does not affect the other.

When two elements are "co-expressed", they are induced at the same time and repressed at the same time. The levels at which two proteins are co-expressed need not been the same for them to be co-expressed.

The term "homozygous" indicates that identical alleles reside at the same loci on homologous chromosomes. In contrast, "heterozygous" indicates that different alleles reside at the same loci on homologous chromosomes. A transgenic animal may be homozygous or heterozygous for a transgene.

The term "luciferase" refers to an enzyme that emits light during the oxidation of its substrate luciferin. The terms luciferin and luciferase do not refer to a particular luciferin or luciferase. They are generic terms for a substrate and its associated enzyme (or protein) that catalyzes a light-producing reaction.

The phrase "*in vivo* imaging" as used herein refers to methods of detecting the presence of an optically detectable protein in whole, live animal. Optically detectable proteins such as fluorescent proteins and luciferases may be detected by *in vivo* imaging. *In vivo* imaging may be used provide 2-D as well as 3-D images of an animal. Charge-coupled device cameras, CMOS, or 3D tomographers may used to carry out *in vivo* imaging.

The term "induced" with respect to a promoter, is intended to encompasses both the initiation of transcription of a downstream nucleic acid, as well as an increase in the rate of transcription of a downstream nucleic acid that is already being transcribed, compared to an uninduced state.

The term "endogenous" with reference to a gene, indicates that the gene is native to a cell, i.e., the gene is present at a particular locus in the genome of a non-modified cell. An endogenous gene may be a wild type gene present at that locus in a wild type cell (as found in nature). An endogenous gene may be a modified endogenous gene if it is present at the same locus in the genome as a wild type gene. An example of such a modified endogenous gene is a gene into which a foreign nucleic acid is inserted. An endogenous gene may be present in the nuclear genome, mitochondrial genome etc.

The term "biologically active", with respect to a gene or protein, refers to a gene or protein that has a biological activity. So-called "knock-outs" are not biologically active. If an altered gene or protein is referred to as being biologically active, then it has the same activity of an unaltered version of the gene or protein. No significant change in phenotype may be observed in an animal having biologically active altered gene relative to an unaltered gene.

The term "ES cell" as used herein refers to pluripotent embryonic stem cells and to such pluripotent cells in the very early stages of embryonic development, including but not limited to cells in the blastocyst stage of development.

The term "construct" refers to a recombinant nucleic acid, generally recombinant DNA, that has been generated for the purpose of the expression of a specific nucleotide sequence(s), or is to be used in the construction of other recombinant nucleotide sequences. A construct might be present in a vector or in a genome.

The term "recombinant" refers to a polynucleotide or polypeptide that does not naturally occur in a host cell. A recombinant molecule may contain two or more naturally-occurring sequences that are linked together in a way that does not occur naturally. A recombinant cell contains a recombinant polynucleotide or polypeptide.

The term "selective marker" refers to a protein capable of expression in a host that allows for ease of selection of those hosts containing an introduced nucleic acid or vector. Examples of selectable markers include, but are not limited to, proteins that confer resistance to antimicrobial agents (e.g., hygromycin, bleomycin, or chloramphenicol), proteins that confer a metabolic advantage, such as a nutritional advantage on the host cell, as well as proteins that confer a functional or phenotypic advantage (e.g., cell division) on a cell.

The term "expression", as used herein, refers to the process by which a polypeptide is produced based on the nucleic acid sequence of a gene. The process includes both transcription and translation.

The term "introduced" in the context of inserting a nucleic acid sequence into a cell, means "transfection", or 'transformation" or "transduction" and includes reference to the incorporation of a nucleic acid sequence into a eukaryotic or prokaryotic cell wherein the nucleic acid sequence may be incorporated into the genome of the cell (e.g., chromosome, plasmid, plastid, or mitochondrial DNA), converted into an autonomous replicon, or transiently expressed (e.g., transfected mRNA).

"Treating" or "treatment" of a condition or disease includes providing a clinical benefit to a subject, and includes: (1) preventing at least one symptom of the conditions, i.e., causing a clinical symptom to not significantly develop in a mammal that may be exposed to or predisposed to the disease but does not yet experience or display symptoms of the disease, (2) inhibiting the disease, i.e., arresting or reducing the development of the disease or its symptoms, or (3) relieving the disease, i.e., causing regression of the disease or its clinical symptoms.

The term "candidate agents" means oligonucleotides, polynucleotides, siRNA, shRNA genes, gene products, polypeptides, small molecules, e.g., up to 2500 Dalton (Da) in size, and pharmacological compounds that are combined with the cells or the animals described herein to screen for their effect on muscle atrophy. In certain cases, a candidate agent may be delivered as a nucleic acid that is transcribed and/or translated to provide the candidate agent, for example, a RNAi molecule or a polypeptide.

The term "coding sequence" refers to a nucleic acid sequence that once transcribed and translated produces a protein, for example, *in vivo*, when placed under the control of appropriate regulatory elements. A coding sequence as used herein may have a continuous ORF or might have an ORF interrupted by the presence of introns or non-coding sequences. In this embodiment, the non-coding sequences are spliced out from the pre-mRNA to produce a mature mRNA.

The term "interrupted coding sequence" refers to a coding sequence comprising a interrupting nucleic acid sequence that prevents the production of the protein encoded by the coding sequence. This interrupting nucleic acid sequence cannot be spliced-out from the pre-mRNA. Excision of this interrupting nucleic acid sequence results in the production of the protein encoded by the coding sequence.

The phrase "muscle cell", as used herein, refers to muscles cells of all kinds, such as, mature muscle cells, including skeletal, smooth and cardiac, precursors of these muscle cells, any intermediate cell existing during the differentiation of a muscle precursor cell, muscle fibers, muscle cell lines, etc. Examples of muscle cells include myoblasts, myotubes, myocytes, cardiac muscle cells, skeletal muscle cells, myofibers etc. Muscle cell may be present *in vivo* (in an animal) or *in vitro* (in a cell culture).

The phrase "secreted reporter enzyme" refers to an enzyme that is secreted from the cell producing it and that enzyme serves as a reporter in that it is detectable by an enzyme assay of, for example, cell culture medium, body fluids, such as, blood, lymph, etc.

The phrase "translational shunt sequence" refers to a nucleic acid sequence which codes for a peptide that provides for separation of two proteins joined by the peptide. Translational shunt sequences are found in a variety of organisms, such as viruses. Translational shunt sequences are useful for generating separate proteins co-translationally, i.e., translational shunt sequences encode peptides that provide for production of two proteins from a single mRNA. A translational shunt sequence is usually cloned in frame between two ORFs, encoding two proteins or polypeptides, forming a single ORF. Translational shunt sequences have been described in Trichas G. et al., BMC Biology, 6: 40, 2008; de Felipe P., Genet. Vaccines Ther. 2(1): 13, 2004; and El Amrani A. et al., Plant Physiol. 135(1):16-24, 2004, which are incorporated by reference for this disclosure.

The phrase "atrogen gene" refers to a gene whose expression is induced in muscle cells of a rodent exposed to an atrophy-inducing stimulus (e.g., fasting, etc) prior to a detectable muscle atrophy phenotype i.e., a detectable loss of muscle mass, shriveling of cells, etc., is observable.

The phrase "atrogen promoter" refers a promoter that is induced in muscle cells of a rodent exposed to an atrophy-inducing stimulus (e.g., fasting, etc) prior to a detectable muscle atrophy phenotype i.e., a detectable loss of muscle mass, shriveling of cells, etc., is observable. An atrogen promoter may be the promoter of a wild type atrogen gene, or an active variant thereof that is, for example, at least 95% identical to a wild type atrogen promoter.

The terms "muscle cell atrophy", "muscle atrophy" and "atrophy" are used interchangeably to refer to muscle cell atrophy *in vitro* or *in vivo.* Muscle cell atrophy, *in vivo*, refers to a decrease in the mass of a muscle, fiber size, cross-sectional area, etc. A decrease in the mass of the muscle is usually accompanied with a weakening of the muscles. Muscle cell atrophy, *in vitro,* refers to muscle cell shriveling, muscle cell death, etc. The terms "muscle cell atrophy", "muscle atrophy" and "atrophy" are used to refer to atrophy caused by a variety of stimuli, unless stated otherwise. Thus, muscle atrophy refers to atrophy due to fasting, cachexia, diabetes, etc.

### DESCRIPTION OF EXEMPLARY EMBODIMENTS

Before the present subject invention is described further, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range is encompassed within the invention.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described. All publications mentioned herein are to disclose and describe the methods and/or materials in connection with which the publications are cited.

It must be noted that as used herein and in the appended claims, the singular forms "a", "and", and "the" include plural referents unless the context clearly dictates otherwise. Thus, for example, reference to "a cell" includes a plurality of cells and reference to "a candidate agent" includes reference to one or more candidate agents and equivalents thereof known to those skilled in the art, and so forth. It is further noted that the claims may be drafted to exclude any optional element. As such, this statement is intended to serve as antecedent basis for use of such exclusive terminology as "solely", "only" and the like in connection with the recitation of claim elements, or use of a "negative" limitation.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

All publications and patents cited in this specification are to disclose and describe the methods and/or materials in connection with which the publications are cited. The citation of any publication is for its disclosure prior to the filing date and should not be construed as an admission that the present invention is not entitled to antedate such publication by virtue of prior invention. Further, the dates of publication provided may be different from the actual publication dates which may need to be independently confirmed.

As will be apparent to those of skill in the art upon reading this disclosure, each of the individual embodiments described and illustrated herein has discrete components and features which may be readily separated from or combined with the features of any of the other several embodiments. Any recited method can be carried out in the order of events recited or in any other order which is logically possible.

### Animal Model for Muscle Cell Atrophy

As noted above, a non-human animal model for muscle cell atrophy is provided comprising a nuclear genome comprising a biologically active atrogen gene in operable linkage with a reporter construct comprising: i. a first coding sequence for a fluorescent or luminescent detectable protein; and ii. a second coding sequence for a secreted reporter enzyme. The atrogen gene, the optically detectable protein and the secreted reporter enzyme are co-expressed in that initiation of muscle cell atrophy results in production of the secreted reporter enzyme and the detectable protein by the cell.

In certain embodiments, the animal may contain a cell comprising a bi-functional reporter gene whose expression changes upon initiation of muscle cell atrophy. In certain cases the reporter construct comprises an atrogen promoter, e.g., the promoter of an atrogen gene, that is operably linked to a first coding sequence for first optically detectable protein and a second coding sequence for a secreted reporter enzyme. The reporter construct may be integrated into the genome of the cell at a specific site or via a random integration event. In certain cases and as will be described in greater detail below, the promoter of the reporter construct may be an endogenous promoter of the host cell, in which case the cell may be made by integrating a nucleic acid containing an IRES into the 3' UTR of an atrogen gene or by modifying an atrogen gene so that it encodes a functional protein and a dual-functional reporter separated by a translational shunt that is endogenous or not endogenous to the cell. In other embodiments, the reporter construct, including the promoter and coding sequences, may be made by recombinant methods outside of the cell and introduced into the cell. In these embodiments, the resultant cell may have two copies of an atrogen promoter (an endogenous atrogen promoter that is part of an unaltered endogenous atrogen gene and a second atrogen promoter that is operably linked to the reporters. As noted above and in particular embodiments, the reporter construct contains an IRES and is present in the 3'UTR of an atrogen gene.

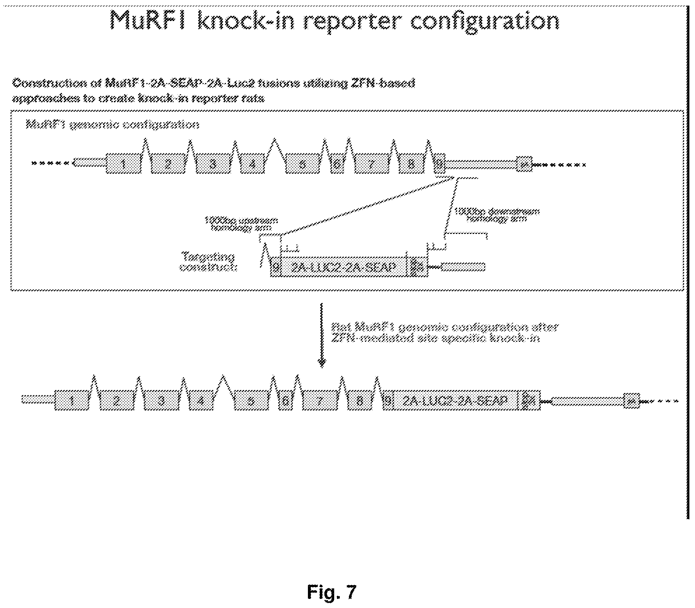

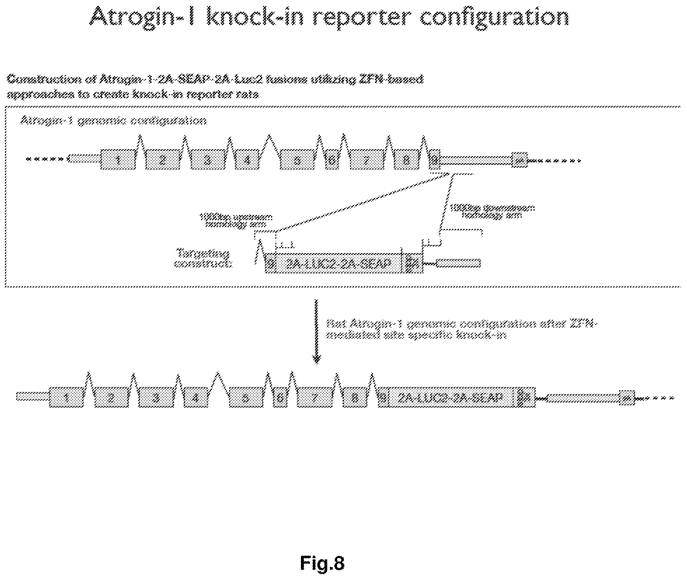

In the embodiment shown in Figs. 7 and 8, exmplary knock-in configurations are described. In these configurations, a reporter cassette is inserted at the 5' end of the coding sequence of an endogenous atrogen gene (e.g., MurRF1 (Fig. 7) or Atrogin-1 (Fig. 8) to produce a fusion in which the coding sequence for the atrogen gene is fused, in frame, with a bi-functional reporter, and the coding sequences for the atrogen gene, the optically detectable reporter (luciferase) and the secretable reporter enzyme (SEAP) are separated by a translational shunt. In this embodiment, induction of the atrogen gene produces a single transcript, and the three proteins (a biologically active atrogen protein containing a translational shunt sequence at its C-terminus, the optically detectable protein containing a translational shunt sequence at its C-terminus, and the secretable reporter enzyme) are translated therefrom. The expression of all products is driven by the atrogen gene promoter.

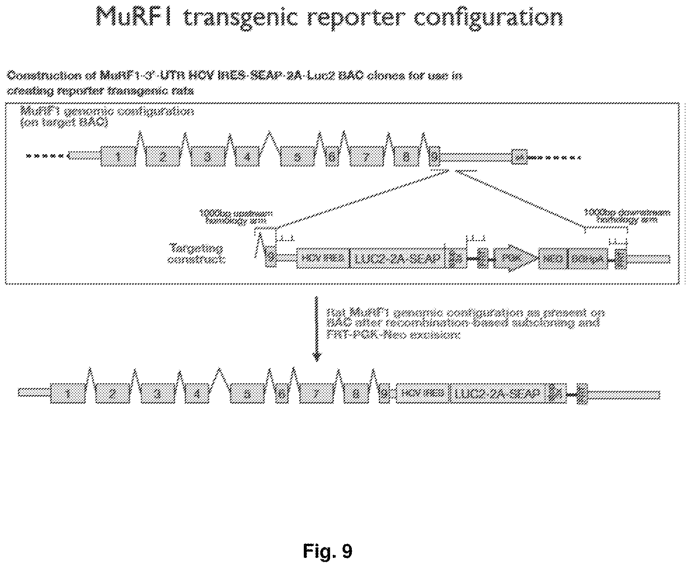

In the embodiment shown in Fig. 9, for example, a transgenic configuration is described in which a BAC (from the same species as the animal into which the BAC is to be introduced) containing an IRES reporter construct is inserted into the 3' UTR of the selected atrogen gene (in this case, MuRF1) is first constructed in E. coli. Once, constructed, the BAC is transferred into an animal to make a transgenic animal. In these transgenic embodiments, the BAC may be integrated into the animal's genome either to replace the endogenous atrogen gene at a different locus to the atrogen gene. As shown in Fig. 9, the 3' UTR of the atrogen gene contains a reporter cassette that employs an IRES. In this embodiment, induction of the atrogen gene produces a single transcript, and the three proteins (a biologically active atrogen protein, the optically detectable protein containing a translational shunt sequence at its C-terminus, and the secretable reporter enzyme) are translated therefrom.

The reporter proteins are operably linked to the atrogen promoter and thus the expression of the reporter protein provides a read-out for the expression from the atrogen gene. In some embodiments, the expression of the secreted reporter enzyme may be determined by monitoring the blood of the animal or the cell culture medium. In some embodiments, the optically detectable protein may be monitored by *in vivo* imaging or by fluorescence microscopy. The animal model provides for non-invasive methods for monitoring initiation and/or progression of muscle cell atrophy. In some embodiments, the change in the expression of the reporter protein is observable even before significant changes in muscle mass or over all cell morphology is evident. Thus, the animal model and cell provided herein may be used to monitor the initial phase of muscle cell atrophy before a phenotype is observable. The animal model and cell are described in more detail below.

The atrogen promoter of the construct may have a wild type nucleotide sequence (i.e., may have a nucleotide sequence that is identical to the promoter of a wild type atrogen gene) or it may have a promoter that has a nucleotide sequence that is at least 90% identical (e.g., at least 95%, at least 98% or at least 99% identical) to a wild type promoter. Given the public availability of the nucleotide sequences of atrogen genes from many different mammalian species, such variants are readily designable.

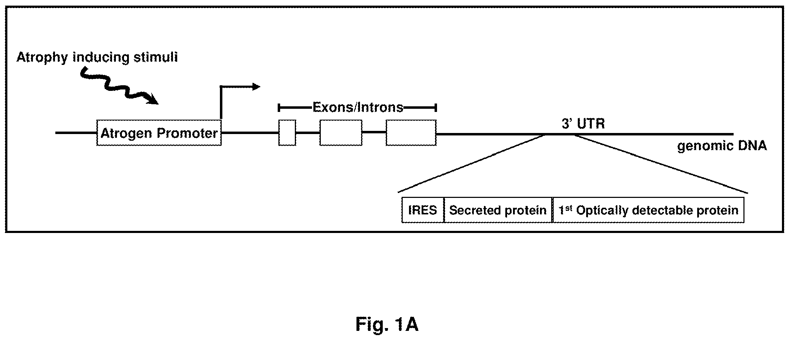

Some of the general principles of the subject animal model, e.g., the promoters, reporters, atrogen genes, shunt sequences, optional sequences, recombinase-encoding sequences, etc., are described below in the context of a so-called "knock-in" animal. As would be readily apparent, those principles are readily applicable to an animal that is not a knock-in animal, i.e., a transgenic animal comprises a genome comprising a reporter construct containing an atrogen promoter operably linked to a first coding sequence for a secreted reported enzyme and a second coding sequence for an optically detectable protein, where reporter construct, including the promoter, was introduced into the cell and is integrated into the cell at a random or pre-determined site that is different to the locus of the endogenous atrogen gene. Fig. 1A illustrates an embodiment of the genomic region of a cell provided herein. With reference to Fig. 1A, this cell comprises an atrogen gene comprising an atrogen promoter, coding sequences for the atrogen protein, and a 3'UTR region. A reporter construct may be present in the 3'UTR of the atrogen gene in some embodiments. In other embodiments, the reporter construct may be present at the 3' end of the coding sequence of the atrogen gene.

The reporter construct comprises a first coding sequence for an first detectable protein and a second coding sequence for a secreted reporter enzyme. In certain embodiments, the reporter construct is operably linked to the promoter of the atrogen gene by virtue of its location in the 3'UTR of the atrogen gene. In other embodiments, the atrogen promoter may be directly linked to the coding sequence of the reporters. In other embodiments, the atrogen promoter may be operably linked to the coding sequence of the reporter proteins by means of a translational shunt sequence at the 3' end of the coding sequence for the atrogen gene. Accordingly, activation of the atrogen promoter results in the production of the protein encoded by the atrogen gene as well as the secreted reporter enzyme and the first optically detectable protein. The reporter construct may contain additional nucleic acid sequences, such as, sequences that may augment the production of the secreted reporter enzyme and the first optically detectable protein. An example of such a sequence is an IRES sequence which provides for translation from a coding sequence(s) operably linked to the IRES sequence. Initiation of muscle cell atrophy may result in the induction of the atrogen promoter leading to the expression of the atrogen gene operably linked to the promoter as well as the proteins encoded by the coding sequences of the reporter construct. Thus, the initiation of muscle cell atrophy may be monitored by measuring activity of the secreted reporter enzyme, for example, in body fluids, such as blood, and/or by the expression of the first optically detectable protein in muscle cells. In some embodiments, the animal model and cell described herein may be used to monitor initiation of muscle cell atrophy. In some embodiments, the animal model and cell described herein may be used to monitor progression of muscle cell atrophy.

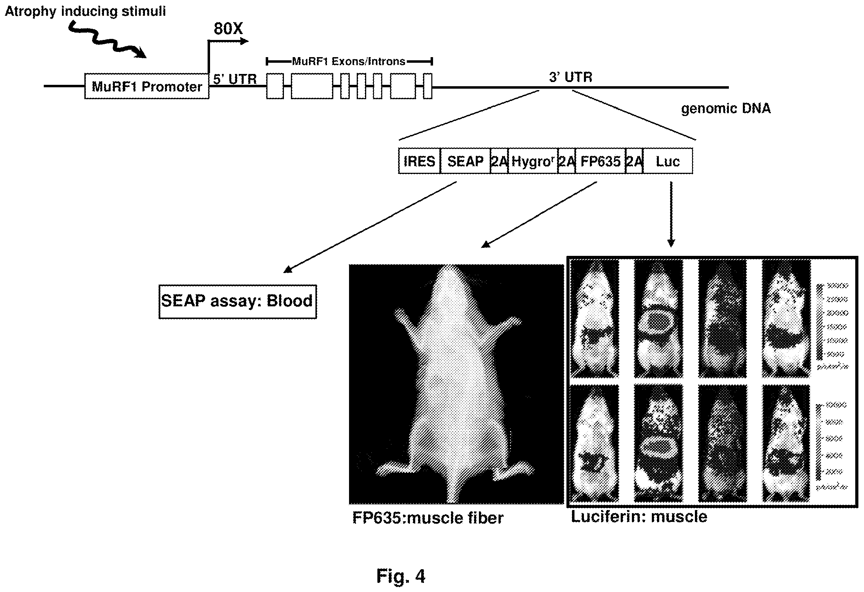

In certain embodiments, the reporter construct further comprises a third coding sequence. The third coding sequence may encode a second optically detectable protein, for example. The second optically detectable protein may provide an additional way of monitoring initiation and/or progression of muscle cell atrophy. A schematic of an example of this embodiment is provided in Fig. 4. The third coding sequence is also operably linked to the atrogen promoter, such that activation of the atrogen promoter results in the production of the protein encoded by the third coding sequence.

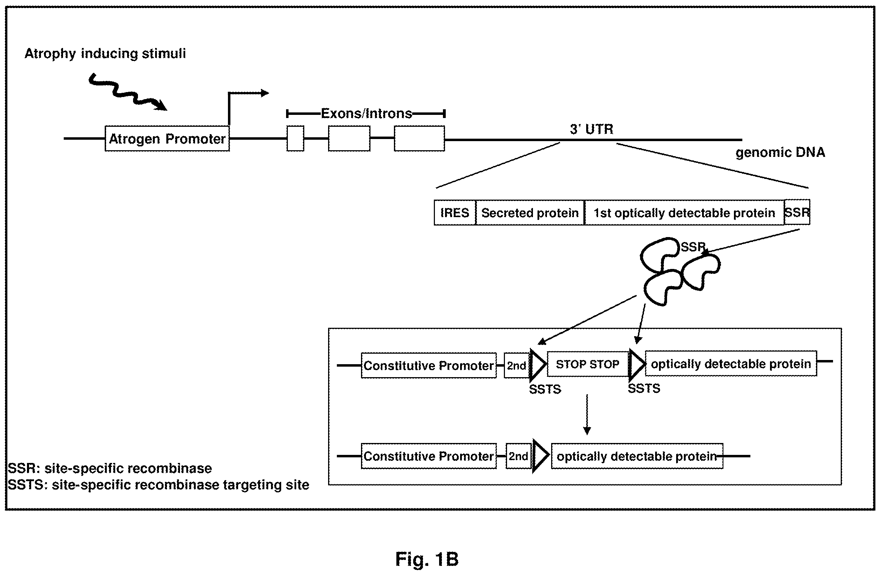

In other embodiments, the third coding sequence may encode a site-specific recombinase. One example of this embodiment is schematically illustrated in Fig. 1B. The initiation of muscle atrophy results in production of the site-specific recombinase. Numerous site-specific recombinases, such as CRE recombinases, are known to one of skill in the art. With reference to Fig. 1B, the cell may further comprise an interrupted coding sequence for a second optically detectable protein operably linked to a constitutively active promoter, such as a CMV promoter. The interrupted coding sequence comprises an interrupting sequence flanked by site-specific recombinase targeting sites. As a consequence of the presence of the interrupting sequence, in this case, termination/STOP codon, the interrupted coding sequence does not encode the second optically detectable protein. Activation of the atrogen promoter leads to the production of the site-specific recombinase, which deletes the interrupting sequence. Thus, cells in which muscle cell atrophy is initiated become marked by the expression of the second optically detectable protein. Since this second optically detectable protein is expressed from a constitutively active promoter, muscle cells undergoing atrophy become permanently marked by the expression of this protein.

In some embodiments, the coding sequences present in the reporter construct are separated by sequences that provide for separation of the proteins encoded by the coding sequences. For example, when the coding sequences form a single ORF, a translational shunt sequence or a nucleic acid sequence coding for a protease cleavage site may be present, in frame, at the end of the first coding sequence and the beginning of the next coding sequence. Translation from this single ORF would result in the production of a polyprotein, i.e., in this example, two proteins linked by a protease cleavage site. These two proteins would be separated by cleavage by a compatible protease. In embodiments where a translational shunt sequence is present between two coding sequences, separate proteins would be produced during translation.

In an embodiment where a translational shunt sequence is present between the first and second sequence, the first coding sequence may be linked to, for example, an IRES sequence or another translational shunt sequence. In an alternative embodiment, each of the coding sequence may be operably linked to, for example, an IRES sequence.

The reporter construct introduced into the cell by any known method, e.g., by transfection by a viral vector, e.g., a lentiviral or retroviral vector. In certain cases, the construct may be inserted into the atrogen gene via homologous recombination. In certain cases, the presence of the reporter construct does not disrupt the cell's ability to atrophy in response to an atrophy-inducing stimulus. In certain embodiments, the reporter construct in the atrogen gene does not effect the expression and/or stability of the protein encoded by the atrogen gene. In some cases, a non-modified cell and a cell in which a reporter construct is present atrogen gene may be substantially similar in terms of expression levels of the protein encoded by the atrogen gene and/or in terms of their response to conditions that stimulate muscle atrophy. In particular examples, a transgenic animal comprising the subject cell, described above, may be indistinguishable from a wild type animal in terms of the expression of the protein encoded by the atrogen gene and in terms of its muscle atrophy phenotype. In certain cases, the reporter construct may optionally retain the coding sequence for the selectable marker used for targeting the reporter construct to the atrogen gene.

Atrogen genes useful in certain embodiments disclosed herein include genes that are expressed upon initiation of muscle atrophy prior to a detectable atrophy-induced phenotype. In some embodiments, the atrogen genes are genes that are expressed upon initiation of muscle atrophy induced by a variety of stimuli, including but not limited to, fasting, cancer cachexia, diabetes, glucocorticoid exposure, renal failure, etc. Thus, in some embodiments, the atrogen gene is a universal atrogen gene as it is common to muscle atrophy induced by a variety of different stimuli. In certain examples, the atrogen gene is specific to muscle atrophy induced by a particular stimuli, for example, the atrogen gene is expressed only in muscle atrophy caused by fasting but not in muscle atrophy caused by cancer, renal disease, etc.

In certain cases, the expression of the atrogen gene is induced at the beginning of the muscle atrophy program, prior to ubiquitin proteosome-dependent muscle proteolysis, which may be assessed by evaluating cell morphology, muscle mass, fiber cross section area, protein content, or gene expression, etc. In certain cases, an atrogen gene may be characterized as having an encoded protein or mRNA that is: a) induced at least 6 hours, at least 12 hours, at least 24 hours, at least 30 hours, or more, before a muscle atrophy phenotype, such as loss of muscle weight, shriveling of muscle cells, decrease in fiber size, etc., is detectable in a rodent, b) induced by at least 2 times, 5 times, 10 times, 20 times, 50 times, 100 times, or more, compared to a control that is not exposed to atrophy stimulator conditions, c) induced through atrophy, and d) contributes to muscle cell atrophy, etc. Exemplary genes that are not considered atrogen genes are genes that are induced by at most 2 times, or at most 3 times, or at most 4 times, or less, during muscle atrophy. For example, in certain cases a gene encoding polyubiquitin that is induced by 2-4 times, or a gene encoding E2₁₄ₖ that is induced 2-3 times, during muscle atrophy, may not be considered an atrogen gene

In certain embodiments, the atrogen gene may encode an E3 ubiquitin ligase. In particular embodiments, the atrogen gene may be the Ub ligase (E3) Atrogin-1, also named Muscle Atrophy F-box (MAFbx). In certain embodiments, the atrogen gene may be Muscle RING Finger 1 (MuRF1), which is also an E3 ligase. MAFbx and MuRF1 were identified as universal markers/mediators of muscle atrophy as the expression of these genes was up-regulated in a variety of animal models of muscle atrophy and animals lacking a functional MAFbx or MuRF1 gene were resistant to denervation induced muscle atrophy (Bodine S.C. et al., Science 294, 1704-1708, 2001, Gomes M.D. et al., Proc. Natl. Acad. Si. USA 98, 14440-14445, 2001). These genes are up-regulated early during the atrophy process and at least in the fasting induced animal model of atrophy, the increase in MAFbx precedes loss of muscle weight.

In certain embodiments, the atrogen gene may be metallothionein-1L, metallothionein-1B, or FoxO1. These genes, in addition to playing a role in initiating muscle cell atrophy may also play a role in maintenance of muscle in an atrophied state. These genes are described in, e.g., Sacheck J.M. et al., FASEB J., 21, 140-155, 2007. In certain aspects, the atrogen gene may be lipin, TG interacting factor, or AMP deaminase. These genes are up-regulated at the initiation of the muscle cell atrophy program.

These and other atrogen genes are been described in a number of research publications and reviews, for example, in Sacheck J.M. et al., FASEB J., 21, 140-155, 2007; Lecker S.H. et al. FASEB J., 18, 39-51, 2004; Jagoe R.T. et al. FASEB J. 16, 1697-1712, 2002, Gomes M.D. et al., Proc. Natl. Acad. Sci. USA 98, 14440-14445, 2001; Bodine S.C. et al., Science 294, 1704-1708, 2001, etc.

Further atrogen genes may be identified by methods well known to a person of skill in the art. For example, cDNA microarrays may be used to compare changes in expression of specific mRNAs in muscles atrophying from different causes to identify genes whose expression changes in muscle atrophying from different causes, as well as to identify genes whose expression changes only in muscle atrophying from a particular cause. In order to identify atrogen genes whose expression change precedes muscle cell atrophy, the mRNA may be collected from muscle cells relatively early after inducing muscle atrophy, such as, within 12hrs, 24hrs, 48hrs, 60 hrs, 72hrs. Alternatively, the optimal time for collecting mRNA may be determined empirically, for example, by ascertaining when a muscle cell atrophy phenotype, such as, decrease in muscle mass, fiber-size, fiber-cross sectional area, becomes evident. Various ways to induce muscle atrophy, as well as various models of muscle atrophy are known and are described in more detail in later sections.

In some embodiments, the reporter construct may be present in two endogenous atrogen genes, where both atrogen genes are up-regulated upon initiation of muscle cell atrophy. In certain other embodiments, two or more copies of a reporter construct may be present in the 3'UTR region of an atrogen gene. In certain aspects, a reporter construct may be present in the 5'UTR of an atrogen gene. In certain embodiments, an atrogen gene may be a wild-type or naturally occurring atrogen gene. In certain embodiments, the atrogen gene may be modified, for example, by a mutation, such as substitution, deletion, addition of one or more nucleotides. The sequences of these genes, including the 3'UTRs, are available at a number of public databases such NCBI, Rat Genome database, Ensembl etc. In instances where the 3'UTR of an atrogen gene has not been characterized, one of skill in the art is aware of numerous methods for identifying the 3'UTR of a gene, for example, by sequencing of RT-PCR product of mRNA of an atrogen gene.

In certain cases, the reporter construct is inserted into the atrogen gene such that the reporter construct is operably linked to the atrogen promoter. In certain embodiments, the reporter construct may also include an IRES or some form of translational re-initiation that would allow for translation from a region of the messenger RNA (mRNA) following a stop codon. In certain other embodiments, the reporter construct may be inserted in frame with the ORF of the atrogen gene, for example, by deleting the stop codon of the atrogen gene. In this case, a sequence that would provide for 5'cap independent translation is not required because the mRNA would have a single open reading frame which would encode a polyprotein. In such a case, inclusion of a protease cleavage site or a translational shunt sequence between the codon encoding the last amino acid of the atrogen protein and the codon encoding the first amino acid of the secreted reporter enzyme provides for separation of the atrogen protein from the secreted reporter enzyme. In certain embodiments each of the coding sequences present in the reporter construct terminate in a stop codon. In this case, an IRES sequence may be included between each coding sequence to provide for translational initiation.

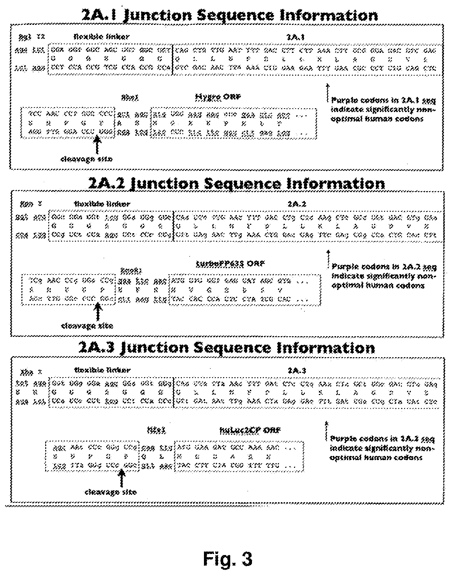

As noted above, in some cases, a reporter construct may comprise a translational shunt sequence present between two coding sequences. Translational shunt sequences are well known in the art and are discussed in a number of references, e.g., Trichas G. et al., BMC Biology, 6: 40, 2008, de Felipe P., Genet. Vaccines Ther. 2(1): 13, 2004, El Amrani A. et al., Plant Physiol. 135(1):16-24, 2004, etc. Translational shunt sequences provide a way to separate several proteins encoded from a single mRNA, for example, in a polycistronic construct. Translational shunt sequences such as those encoding 2A peptides result in the cotranslational "cleavage" of proteins. Translational shunt sequences have been demonstrated to work in a number of eukaryotic cells as well as in transgenic mammals. An example of translational shunt sequences is provided in Fig. 3.

In some embodiments, the reporter construct may comprise a first coding sequence for a first optically detectable protein and a second coding sequence for a secreted reporter enzyme. A number of secreted reporter enzymes, such as, secreted alkaline phosphatase (SEAP), may be used. An enzyme that may be modified, for example, by addition of a secretion signal peptide and assayed by a reliable assay may be used. SEAP has been extensively used to measure promoter/enhancer activity and has been used as an *in vivo* reporter, see, e.g., Gene 66, 1-10, 1988, Mets. Enzymol. 216, 362-368, 1992, Nilsson E.E., et al., Cancer Chemother. Pharmacol., 49: 93-100, 2002, Westfall S.D. and Skinner M.K., Mol Cancer Ther 2005, 4(11), etc. Kits and instructions for measuring SEAP are available, for example, from Applied Biosystems, Clontech, etc.

In the invention, the first optically detectable protein is a fluorescent protein or a luciferase. In some cases, the reporter construct may further comprise a third coding sequence for a second optically detectable protein, wherein initiation of muscle atrophy results in production of the protein. In this embodiment, any combination of first and second optically detectable protein may be used, provided that the two proteins are optically distinguishable. Thus, for example, the first optically detectable protein may be a first fluorescent protein and the second optically detectable protein may be a second fluorescent protein that is optically distinguishable from the first fluorescent protein, for example, green fluorescent protein and red fluorescent protein. In other embodiments, the first optically detectable protein may be a fluorescent protein and the second optically detectable protein may be a luciferase, or *vice versa*, where the optical signal produced by luciferase activity is distinguishable from the optical signal produced by the fluorescent protein, for example, fire fly luciferase and red fluorescent protein. In alternative embodiments, the first and the second optically detectable proteins may be luciferases that are optically distinguishable, for example, firefly luciferase and click beetle luciferase. Non-limiting examples of fluorescent proteins include mPlum, mCherry, tdTomato, mStrawberry, mOrange, yellow fluorescent protein, green fluorescent protein, red fluorescent protein, mCitrine, Venus and YPet, cyan fluorescent protein, T-sapphire, Katusha, DsRed-Express, far-red fluorescent protein FP635, etc.

Luciferase used in the present disclosure may be derived from the renilla (protein accession no. CAA01908), firefly (protein acc. No. CAA59282), click-beetle (protein acc. No. AAQ19142), G. princes luciferase (GLUC), or other sources of luciferase. Luciferase used in the present disclosure cover mutated and otherwise modified versions such as mammalian codon optimized luciferases. Methods for using luciferases for real-time imaging of luciferase expression in live animals have been described (L. F. Greer, et al., Luminescence 17: 43-74, 2002). Luciferase, from various sources, has been used in various assays and reporting capacities. For example, U.S. Pat. No. 6,387,675 discloses the use of the luciferase gene of click beetle, *P. plagiophthalamus,* in eukaryotic cells for biosensing.

In some embodiments, the reporter construct may be inserted into a genome, e.g., the endogenous atrogen gene via homologous recombination. Methods for carrying out homologous recombination are well known in the art. In such methods, a targeting vector is constructed and introduced into a cell of interest. The vector usually contains of an insertion cassette of an exogenous nucleic acid sequence which is to be inserted into the genome and a positive selectable marker. This insertion cassette is flanked by regions of homology to the target gene. The vector also has a negative selectable marker, located outside the regions of homology. The regions of homology are genetic sequences complementary to specific sites on the genome where targeting is desired. The vector hybridizes to genomic DNA at these regions of homology, and in a small percentage of cells there is a double crossover recombination between the genomic DNA and the vector. Such a crossover exchanges genetic information inside of the regions of homology, substituting the insertion cassette for the target genomic region. This technology takes advantage of the process of recombination to swap the endogenous sequence for the exogenous one. The construct may be integrated into the genome of the cell using any method. In certain embodiments, zinc-finger nucleases may be employed, as described in Geurts et al (Knockout rats via embryo microinjection of zinc-finger nucleases. Science. 2009 325:433); Durai et al (Zinc finger nucleases: custom-designed molecular scissors for genome engineering of plant and mammalian cells. Nucleic Acids Res. 2005 33:5978-90) and Porteus et al (Gene targeting using zinc finger nucleases. Nat Biotechnol. 2005 23:967-73).

The subject cell, described above, may be an ES cell, a myoblast, a myotube, muscle fiber, etc. A number of cell lines as well as methods for introducing nucleic acid into cells are known in the art. The stem cells may be obtained from any mammalian species, e.g. human. In exemplary embodiments, a muscle cell line may be used, such as, SM3, Aza2, BC3H-1, BD1, BD2, BD10, C2C12, TD33, TD38, TD45, TG1, C2, HL-1, AT-1, etc.

A subject cell may be cultured *in vitro,* under appropriate culture conditions. In some embodiments, the cell may be cultured *in vivo*, for example in a particular organ, tissue in an animal. In certain embodiments, the cell may be cultured *ex vivo*, for example in a particular organ, tissue of an animal. In other embodiments, the cell may be implanted in a particular region of an animal, e.g., in a skeletal muscle, producing a transgenic animal that comprises cells comprising a subject reporter construct which may, in certain embodiments, be present in atrogen gene.

The animal model may be mammalian, such as, equine, bovine, porcine, canine, feline, rodent, etc. In some embodiments the mammal is a murine, e.g. a mouse or rat.

In some embodiments, the animal model may be generated from an ES cell. In this embodiment, all cells of the animal may have the reporter construct. For embryonic stem (ES) cells, an ES cell line may be employed, or embryonic cells may be obtained freshly from a host, *e.g.* mouse, rat, guinea pig, *etc.* Such cells are grown on an appropriate fibroblast-feeder layer or grown in the presence of appropriate growth factors, such as leukemia inhibiting factor (LIF). When ES cells have been transformed, they may be used to produce gene-targeted animals. See U.S. Patents 5,387,742; 4,736,866; and 5,565,186; and Larson et al. (2000) Mol. Ther. 2:631-639 for methods of making gene-targeted animals. After transformation, the cells are plated onto a feeder layer in an appropriate medium. Cells containing the reporter construct may be detected by employing a selective medium. After sufficient time for colonies to grow, they are picked and analyzed for the occurrence of homologous recombination of the construct. Those colonies that are positive may then be used for embryo manipulation and blastocyst injection. Blastocysts are obtained from 4 to 6 week old superovulated females. The ES cells are trypsinized, and the modified cells are injected into the blastocoel of the blastocyst. After injection, the blastocysts are returned to each uterine horn of pseudopregnant females. Females are then allowed to go to term and the resulting litters screened for cells having the construct. By providing for a different phenotype of the blastocyst and the ES cells, chimeric progeny can be readily detected. The chimeric progeny is further breeded to identify those that have germ-line cells having the construct. Animals having a heterozygous alteration are generated by breeding of chimeras. Male and female heterozygotes are typically bred to generate homozygous animals.

In some embodiments, the animal model is generated by implanting a subject cell into an animal. In this case, the animal model is a transgenic animal that comprises cells comprising the reporter construct. The cell may be an ES cell, a myotube, a myoblast, etc. The cell may be implanted to any of the muscles of the animal including but not limited to cardiac muscle, skeletal muscle, etc.

Muscle cell atrophy may be initiated by a number of stimuli including but not limited to fasting, ageing, diabetes, advanced cancer, renal failure, sepsis, cachexia, arthritis, osteoporosis, diabetes, denervation, immobilization, muscle unloading, spinal cord injury, glucocorticoid treatment, and the like. *In vitro,* muscle cell atrophy may be initiated by starving, exposure of cells to for example, glucocorticoids, or to viruses.

### Methods of Screening

A method using the animal model described above for identifying agents that modulate initiation of muscle cell atrophy is provided. In exemplary embodiments, the method involves contacting a subject animal, described above, with a candidate agent, and determining the effect, if any, of the candidate agent on the production of the proteins encoded by the reporter construct. In some embodiments, the method involves contacting an animal model of muscle cell atrophy, comprising the cell, with a candidate agent, and determining the effect, if any, of the candidate agent on muscle cell atrophy in the animal.

The term "agent" as used herein describes any molecule, e.g. protein or non-protein organic or inorganic pharmaceutical. Agents of particular interest are those that inhibit initiation of muscle cell atrophy. A plurality of assays is run in parallel with different agent concentrations to obtain a differential response to the various concentrations. One of these concentrations may serve as a negative control, *i.e.* at zero concentration or below the level of detection.

The terms "candidate agent", "test agent", "agent", "substance" and "compound" are used interchangeably herein. Candidate agents encompass numerous chemical classes, typically synthetic, semi-synthetic, or naturally-occurring inorganic or organic molecules. Candidate agents include those found in large libraries of synthetic or natural compounds. For example, synthetic compound libraries are commercially available from Maybridge Chemical Co. (Trevillet, Cornwall, UK), ComGenex (South San Francisco, CA), and MicroSource (New Milford, CT). Alternatively, libraries of natural compounds in the form of bacterial, fungal, plant and animal extracts are available from Pan Labs (Bothell, WA) or are readily producible.

Candidate agents may be small organic or inorganic compounds having a molecular weight of more than 50 and less than about 2,500 Da. Candidate agents may comprise functional groups necessary for structural interaction with proteins, particularly hydrogen bonding, and may include at least an amine, carbonyl, hydroxyl or carboxyl group, and may contain at least two of the functional chemical groups. The candidate agents may comprise cyclical carbon or heterocyclic structures and/or aromatic or polyaromatic structures substituted with one or more of the above functional groups. Candidate agents are also found among biomolecules including peptides, saccharides, fatty acids, steroids, purines, pyrimidines, derivatives, structural analogs or combinations thereof.

Candidate agents are obtained from a wide variety of sources including libraries of synthetic or natural compounds. For example, numerous means are available for random and directed synthesis of a wide variety of organic compounds and biomolecules, including expression of randomized oligopeptides. Alternatively, libraries of natural compounds in the form of bacterial, fungal, plant and animal extracts are available or readily produced. Additionally, natural or synthetically produced libraries and compounds are readily modified through conventional chemical, physical and biochemical means, and may be used to produce combinatorial libraries. Known pharmacological agents may be subjected to directed or random chemical modifications, such as acylation, alkylation, esterification, amidification, *etc.* to produce structural analogs. New potential therapeutic agents may also be created using methods such as rational drug design or computer modeling.

Screening may be directed to known pharmacologically active compounds and chemical analogs thereof, or to new agents with unknown properties such as those created through rational drug design.

Agents that modulate initiation of muscle cell atrophy may decrease expression of the secreted reporter enzyme and the first optically detectable protein by at least 10%, at least 20%, at least 30%, at least 40%, at least 50%, at least 60%, at least 70%, at least 80%, or at least 90%, or more, relative to controls that have not been exposed to the agent.

Agents that modulate initiation of muscle cell atrophy may be subjected to directed or random and/or directed chemical modifications, such as acylation, alkylation, esterification, amidification, etc. to produce structural analogs. Such structural analogs include those that increase bioavailability, and/or reduced cytotoxicity. Those skilled in the art can readily envision and generate a wide variety of structural analogs, and test them for desired properties such as increased bioavailability and/or reduced cytotoxicity, etc.

### In vivo screening assays

An *in vivo* screening assay is provided. In certain embodiments, the assay comprises identifying agents that alter production of the reporter proteins encoded by the reporter construct present, using an animal model comprising a cell comprising the reporter construct. The method generally involves contacting the animal model with a candidate agent, and determining the effect of the agent on production of the reporter proteins compared with production of the reporter proteins in a control animal not treated with the agent.

The animal model for muscle cell atrophy may be subjected to an atrophy inducing stimuli before or after contacting the animal with a candidate agent. In certain embodiments, the animal model for muscle cell atrophy may be subjected to an atrophy inducing stimuli and contacted with a candidate agent simultaneously.

A number of conditions known to induce atrophy may be used as an atrophy inducing stimuli, including but not limited to, glucocorticoid administration, starvation, cardiac failure, denervation, immobilization, sepsis, implanting hepatoma, streptozotocin-induced diabetes mellitus, nephrectomy, etc.

The animal is contacted with the candidate agent, e.g., the agent is administered to the animal by any acceptable route of administration, including, but not limited to, oral (e.g., oral gavage), intravenous, intramuscular, intranasal, subcutaneous, intragastric, etc., e.g., any enteral or parenteral route. A single dose is administered, or multiple doses over a period of time are administered.

As noted earlier, the production of secreted reporter enzyme may be monitored in body fluids, such as blood. For example, SEAP may be detected and quantitated in blood drawn at certain time points after administration of a candidate agent. SEAP may be monitored by well known methods described in Nilsson E.E., et al., Cancer Chemother. Pharmacol., 49: 93-100, 2002, Westfall S.D. and Skinner M.K., Mol Cancer Ther 2005, 4(11), etc., as well as, by using kits from Applied Biosystems, Clontech, Invivogen, for example.

*In vivo* imaging of fluorescent/luminescent reporter proteins has been used extensively. For example, Burdette J.E. in Journal of Mol. Endocrin., 40, 253-261, 2008, reviews utilizing computed tomography, magnetic resonance imaging, ultrasonography, positron emission tomography, single-photon emission computed tomography, etc., for *in vivo* imaging. Methods for using luciferases for real-time imaging of luciferase expression in live animals have been described (e.g., L. F. Greer, et al., Luminescence 17: 43-74, 2002). *In vivo* imaging of fluorescent proteins in live animals is described in, e.g., Hoffman, Cell Death and Differentiation (2002) 9, 786-789.

The production of reporter protein(s) may be monitored at different points before and after subjecting the animal model to conditions that induce muscle cell atrophy. Similarly, the effect of a candidate agent may be determined by measuring the reporter proteins at several time points. For example, the production of reporter protein(s) may be measured at time 0hrs, 12hrs, 24 hrs, 36 hrs, 48 hrs, 72 hrs, 120 hrs, 1 week, 2 week, 1 month, 2 months, 3 months, 5 months, etc., after contacting the cell with a candidate agent under conditions that induce muscle cell atrophy. In certain embodiments, the measurement of the reporter proteins may be complimented by measuring the expression of the atrogen gene(s), as well as measuring cell/fiber size, morphology, muscle strength, etc.

Agents that modulate initiation of muscle cell atrophy may decrease expression of the secreted reporter enzyme and the first optically detectable protein by at least 10%, at least 20%, at least 30%, at least 40%, at least 50%, at least 60%, at least 70%, at least 80%, or at least 90%, or more, relative to controls that have not been exposed to the agent.

In certain embodiments, a candidate agent found to be effective in decreasing the production of the reporter protein(s), *in vivo* or *in vitro,* may be further assessed by administering it to an animal under conditions that induce muscle cell atrophy. The effectiveness of the candidate compound may be determined by measuring the time of onset of muscle cell atrophy phenotype as well as severity of the muscle cell atrophy. The effectiveness of the candidate compound may be determined by measuring muscle weight, muscle strength, fiber cross sectional area, kinetic experiments, etc.

### In vitro assays

In some cases, *in vitro* methods for identifying agents that modulate initiation of muscle cell atrophy are provided. Cell-based methods generally involve contacting a cell that produces reporter proteins upon initiation of muscle cell atrophy with a candidate agent, and determining the effect, if any, of the candidate agent on the production of the reporter proteins as compared to a control cell not treated with the candidate agent.

The cell may be subject to conditions that induce muscle cell atrophy, prior to, after or simultaneous with contacting the cell with a candidate agent. Conditions that induce muscle cell atrophy *in vitro,* include, starvation, glucorcorticoid exposure, viral infection, hypoxia, etc.

The production of reporter protein(s) may be monitored at different points before and after subjecting the cells to conditions that induce muscle cell atrophy. Similarly, the effect of a candidate agent may be determined by measuring the reporter proteins at several time points. For example, the production of reporter protein(s) may be measured at time 0hrs, 2hrs, 4hrs, 8hrs, 12hrs, 24 hrs, 36 hrs, 48 hrs, 72 hrs, 120 hrs, 1 week, 2 week, and up to 1 month, after contacting the cell with a candidate agent under conditions that induce muscle cell atrophy.

As noted earlier, determining production of secreted reporter enzyme may be by measuring activity of a secreted reporter enzyme in the cell culture medium. For example, when the secreted reporter enzyme is SEAP, its activity may be assayed by kits available from Applied Biosystems or Clontech, etc.

The optically detectable protein may be detected and its level quantitated by, for example, by a microscope, fluorescence activated cell sorter, fluorimeter, etc.

In certain embodiments, the measurement of the reporter proteins may be complimented by measuring the expression of the atrogen gene(s), as well as measuring cell/fiber size, morphology, etc.

### AGENTS

Formulations, including pharmaceutical formulations, comprising an agent identified by a screening method presented herein, are provided. In general, a formulation comprises an effective amount of an agent. An "effective amount" means a dosage sufficient to produce a desired result, e.g., a decrease in production of the secreted reporter enzyme, the first optically detectable protein, MuRF1, or prevent further loss of muscle mass, etc.

### Formulations

In the subject methods, the active agent(s) may be administered to the host using any convenient means capable of producing the desired result. Thus, the agent can be incorporated into a variety of formulations for therapeutic administration. More particularly, the agents of the present invention can be formulated into pharmaceutical compositions by combination with appropriate, pharmaceutically acceptable carriers or diluents, and may be formulated into preparations in solid, semi-solid, liquid or gaseous forms, such as tablets, capsules, powders, granules, ointments, solutions, suppositories, injections, inhalants and aerosols.

In pharmaceutical dosage forms, the agents may be administered in the form of their pharmaceutically acceptable salts, or they may also be used alone or in appropriate association, as well as in combination, with other pharmaceutically active compounds. The following methods and excipients are merely exemplary and are in no way limiting.

For oral preparations, the agents can be used alone or in combination with appropriate additives to make tablets, powders, granules or capsules, for example, with conventional additives, such as lactose, mannitol, corn starch or potato starch; with binders, such as crystalline cellulose, cellulose derivatives, acacia, corn starch or gelatins; with disintegrators, such as corn starch, potato starch or sodium carboxymethylcellulose; with lubricants, such as talc or magnesium stearate; and if desired, with diluents, buffering agents, moistening agents, preservatives and flavoring agents.

The agents can be formulated into preparations for injection by dissolving, suspending or emulsifying them in an aqueous or nonaqueous solvent, such as vegetable or other similar oils, synthetic aliphatic acid glycerides, esters of higher aliphatic acids or propylene glycol; and if desired, with conventional additives such as solubilizers, isotonic agents, suspending agents, emulsifying agents, stabilizers and preservatives.

The agents can be utilized in aerosol formulation to be administered via inhalation. The compounds of the present invention can be formulated into pressurized acceptable propellants such as dichlorodifluoromethane, propane, nitrogen and the like.

Unit dosage forms for oral administration such as syrups, elixirs, and suspensions may be provided wherein each dosage unit, for example, teaspoonful, tablespoonful, tablet, contains a predetermined amount of the composition containing one or more inhibitors. Similarly, unit dosage forms for injection such as intravenous, intramuscular administration may comprise the inhibitor(s) in a composition as a solution in sterile water, normal saline or another pharmaceutically acceptable carrier.

The term "unit dosage form," as used herein, refers to physically discrete units suitable as unitary dosages for human and animal subjects, each unit containing a predetermined quantity of compounds of the present invention calculated in an amount sufficient to produce the desired effect in association with a pharmaceutically acceptable diluent, carrier or vehicle. The specifications for the novel unit dosage forms of the present invention depend on the particular compound employed and the effect to be achieved, and the pharmacodynamics associated with each compound in the host.

Intranasal formulations will usually include vehicles that neither cause irritation to the nasal mucosa nor significantly disturb ciliary function. Diluents such as water, aqueous saline or other known substances can be employed with the subject invention. The nasal formulations may also contain preservatives such as, but not limited to, chlorobutanol and benzalkonium chloride. A surfactant may be present to enhance absorption of the subject proteins by the nasal mucosa.

An agent of the invention can be administered as injectables. Typically, injectable compositions are prepared as liquid solutions or suspensions; solid forms suitable for solution in, or suspension in, liquid vehicles prior to injection may also be prepared. The preparation may also be emulsified or the active ingredient encapsulated in liposome vehicles.

Suitable excipient vehicles are, for example, water, saline, dextrose, glycerol, ethanol, or the like, and combinations thereof. In addition, if desired, the vehicle may contain minor amounts of auxiliary substances such as wetting or emulsifying agents or pH buffering agents. Actual methods of preparing such dosage forms are known, or will be apparent, to those skilled in the art. See, e.g., Remington's Pharmaceutical Sciences, Mack Publishing Company, Easton, Pennsylvania, 17th edition, 1985. The composition or formulation to be administered will, in any event, contain a quantity of the agent adequate to achieve the desired state in the subject being treated.

The pharmaceutically acceptable excipients, such as vehicles, adjuvants, carriers or diluents, are readily available to the public. Moreover, pharmaceutically acceptable auxiliary substances, such as pH adjusting and buffering agents, tonicity adjusting agents, stabilizers, wetting agents and the like, are readily available to the public.

### Dosages

Although the dosage used will vary depending on the clinical goals to be achieved, a suitable dosage range is one which provides up to about 1 µg to about 1,000 µg or about 10,000 µg of an agent that produces a desired result can be administered in a single dose. Alternatively, a target dosage of an agent that produces a desired result can be considered to be about in the range of about 0.1-1000 µM, about 0.5-500 µM, about 1-100 µM, or about 5-50µM in a sample of host blood drawn within the first 24-48 hours after administration of the agent.

Those of skill will readily appreciate that dose levels can vary as a function of the specific compound, the severity of the symptoms and the susceptibility of the subject to side effects. Preferred dosages for a given compound are readily determinable by those of skill in the art by a variety of means.

### Routes of administration

An agent that produces a desired result is administered to an individual using any available method and route suitable for drug delivery, including *in vivo* and *ex vivo* methods, as well as systemic and localized routes of administration.

Conventional and pharmaceutically acceptable routes of administration include intranasal, intramuscular, intratracheal, intratumoral, subcutaneous, intradermal, topical application, intravenous, rectal, nasal, oral and other parenteral routes of administration. Routes of administration may be combined, if desired, or adjusted depending upon the agent and/or the desired effect. The composition can be administered in a single dose or in multiple doses.

The agent can be administered to a host using any available conventional methods and routes suitable for delivery of conventional drugs, including systemic or localized routes. In general, routes of administration contemplated herein include, but are not necessarily limited to, enteral, parenteral, or inhalational routes.

Parenteral routes of administration other than inhalation administration include, but are not necessarily limited to, topical, transdermal, subcutaneous, intramuscular, intraorbital, intracapsular, intraspinal, intrasternal, and intravenous routes, *i.e*., any route of administration other than through the alimentary canal. Parenteral administration can be carried to effect systemic or local delivery of the agent. Where systemic delivery is desired, administration typically involves invasive or systemically absorbed topical or mucosal administration of pharmaceutical preparations.

The agent can also be delivered to the subject by enteral administration. Enteral routes of administration include, but are not necessarily limited to, oral and rectal delivery.

Methods of administration of the agent through the skin or mucosa include, but are not necessarily limited to, topical application of a suitable pharmaceutical preparation, transdermal transmission, injection and epidermal administration. For transdermal transmission, absorption promoters or iontophoresis are suitable methods. Iontophoretic transmission may be accomplished using commercially available "patches" which deliver their product continuously via electric pulses through unbroken skin for periods of several days or more.

A variety of hosts (wherein the term "host" is used interchangeably herein with the terms "subject" and "patient") are treatable according to the subject methods. Generally such hosts are "mammals" or "mammalian", where these terms are used broadly to describe organisms which are within the class mammalia, including the orders carnivore (*e.g.,* dogs and cats), rodentia (*e.g.,* mice, guinea pigs, and rats), and primates (*e.g.*, humans, chimpanzees, and monkeys). In many embodiments, the hosts may be humans.

### System

A system for monitoring initiation and/or progression of muscle cell atrophy is provided. This system may comprise two transgenic animals which may be mated to provide progeny comprising cells comprising a reporter construct which may or may not be present in the 3'UTR of an atrogen gene and an interrupted coding sequence for a second optically detectable protein. The reporter construct, as has been described in some embodiments above, may comprise a first coding sequence for a secreted reporter enzyme, a second coding sequence for a first optically detectable protein and a third coding sequence for a site specific recombinase (SSR). The interrupted coding sequence as described above, cannot encode the second optically detectable protein due to the presence of an interrupting sequence, for example, where the interrupting sequence leads to a premature termination of translation. This interrupting sequence is flanked by site-specific recombinase targeting sites (SSRTS) that are compatible to the site specific recombinase expressed under the control of the atrogen promoter. Initiation of muscle cell atrophy in the progeny results in the production of the SSR which excises the interrupting sequence leaving a single copy of the SSRTS in the coding sequence for the second optically detectable protein. The resulting modified coding sequence can then encode the second optically detectable protein. The modified coding sequence is operably linked to a constitutively active promoter. Thus, once the disrupting sequence has been excised by SSR produced upon initiation of muscle cell atrophy, all the cells in which atrophy was initiated are marked by the production of the second optically detectable protein. Thus, cells in which muscle cell atrophy was induced are permanently marked. The continued production of the second optically detectable protein is thus independent of continuing muscle atrophy unlike the production of the first optically detectable protein, the production of which would decrease upon inhibition of muscle cell atrophy. This marking system provides a way to follow specific muscles during screening for candidate agents that modulate muscle cell atrophy. Examples of animals useful for practicing methods presented herein, atrogen genes, reporter constructs and first and second optically detectable proteins have been discussed in the preceding sections. The constitutive promoter may be a promoter from cytomegalovirus (CMV), mouse mammary tumor virus (MMTV), Rous sarcoma virus (RSV), or adenovirus, etc. Exemplary promoters include the promoter from the immediate early gene of human CMV (Cell 41:521-530, 1985); the promoter from the long terminal repeat (LTR) of RSV (Proc. Natl. Acad. Sci. USA 79:6777-6781, 1982); SV40 early promoter; and the adenovirus major late promoter.

In certain embodiments, the progeny produced from mating the two transgenic animals of this system may be used to screen for agents that modulate muscle cell atrophy. In particular embodiments, the progeny may be used to further characterize the process of muscle atrophy by, for example, imaging the permanently marked muscle cells during the initiation and progression of muscle atrophy. In exemplary embodiments, the expression profile of these permanently marked muscle cells may be determined by isolating these cells from the progeny.

### Utility

The *in vivo* and *in vitro* models presented herein provide for methods to identify and test agents that may decrease muscle cell atrophy. These agents may be used in formulations that may be used to treat subjects with muscle cell atrophy. In addition, these agents may be given prophylactically to subjects at risk for developing muscle cell atrophy. A subject that may benefit from an agent identified by the methods provided herein may have or be at risk for developing muscle cell atrophy caused by a variety of stimuli. These stimuli include but are not limited to fasting, ageing, advanced cancer, renal failure, sepsis, cachexia, arthritis, osteoporosis, and diabetes. Atrophy of muscles may also be a result of their disuse or denervation, e.g., immobilization, muscle unloading, spinal cord injury, etc. In certain embodiments, the subject may have a health problem that is exacerbated by muscle cell atrophy, such as, HIV, chronic heart failure, chronic kidney disease, liver cirrhosis, burn injuries, osteoporosis, arthritis, etc. The methods of using cells and animal models to screen for candidate compounds that inhibit muscle cell atrophy may be used identify agents that improve protein content, fiber diameter, force production, and fatigue resistance of muscles in subjects with muscle cell atrophy. In one embodiment, a compound identified by the method described above may be employed to treat or prevent atrophy of the diaphragm during ventilator support after surgery, which occurs very rapidly.

### EXAMPLES

The following examples are provided in order to demonstrate and further illustrate certain embodiments and aspects of the present invention and are not to be construed as limiting the scope thereof.

### Example 1

### Generation of reporter construct 1

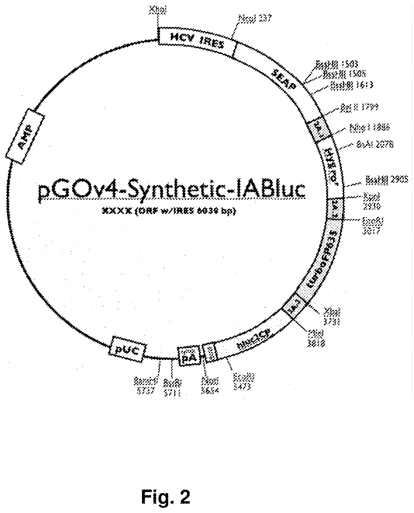

A plasmid containing a reporter construct shown in Fig. 2 is generated by standard molecular biology techniques. The ORF for secreted alkaline phosphatase (SEAP), hygromycin resistance, turboFP635 and luciferase (hluc2CP) are cloned downstream of an HCV IRES sequence, separated by FMDV 2A translational shunt sequences. Translational shunt sequences allow individual ORFs to be resolved without the need for proteases. Junction sequences 2A.1, 2A.2 and 2A.3 with flexible linkers are shown in Fig. 3.

The plasmid shown in Fig. 2 is modified by cloning in flanking mouse genomic sequences and appropriate elements to allow gene knock-in by homologous recombination. Flanking sequences from the 3'UTR region of mouse MuRF1 gene for mediating homologous recombination are cloned in before the HCV IRES sequence and after the synthetic poly A sequence. A nucleotide sequence encoding thymidine kinase is included as a negative selection marker.

The resulting knock-in plasmid is transfected into embryonic stem (ES) cells. Cells that grow in the presence of hygromycin and gancyclovir are selected as cells in which the reporter construct is correctly targeted. Correct targeting of the reporter construct into the 3'UTR region of the MuRF1 gene is confirmed by sequencing.

The knock-in plasmid containing the reporter construct with the flanking sequences from the 3'UTR region of mouse MuRF1 gene and a nucleotide sequence encoding thymidine kinase as a negative selection marker is also transfected into murine myotubes (e.g., C2C12 line) and myoblasts to establish hygromycin and gancyclovir resistant cell lines. Correct targeting of the reporter construct in these cell lines is confirmed by sequencing and by inducing atrophy by contacting these cells with a glucocorticoid. If the reporter construct is correctly targeted, these cells should express MuRF1, SEAP, turboFP635 and luciferase.

### Example 2

### Screening for inhibitors of muscle cell atrophy

The myotube cell line generated in Example 1 is used to screen for candidate compounds that can inhibit muscle atrophy. The myotube cell line containing the reporter construct in the 3'UTR region of MuRF1 gene is contacted with a glucocorticoid, such as dexamethasone, and the levels of SEAP in the culture medium and the expression level of FP635 is measured. Cells that have initiated muscle atrophy show increased expression levels of SEAP and FP635 compared to non-contacted cells. These cells are then contacted with the candidate compounds and the expression levels of the reporters, i.e., SEAP and FP635 is monitored over a period of time. A decrease in the expression levels of the reporters compared to cells contacted with dexamethasone and a negative control compound, such as DMSO, indicates that the candidate compound inhibits muscle atrophy.

### Example 3

### Animal Model for Muscle Cell Atrophy 1

The ES cells with the correctly targeted reporter construct generated in Example 1 are used to generate transgenic mouse lines using standard procedures. The genomic region of cells in mouse lines generated from such ES cells is depicted in Fig. 4. The mouse lines are administered dexamethasone to induce muscle atrophy. Muscles in which atrophy is initiated are monitored by *in vivo* imaging of either the FP635 fluorescent protein or of luciferase activity after administration of luciferin. Initiation of muscle atrophy may also be monitored by measuring levels of SEAP in blood. Fig. 4 shows that a transgenic mouse line generated from the ES cells from example 1 when subjected to a muscle atrophy inducing stimuli, would have detectable levels of SEAP in the blood and would express FP635 in muscle cells in which atrophy is initiated. Additionally, administration of luciferin would lead to detection of luciferase expression in cells in which muscle atrophy is initiated. Fluorescein from FP635 or luminescence from luciferase activity would be detected by *in vivo* imaging of the whole animal as shown in Fig. 4. Measurements may be taken at several time points in the same animal as these methods do not require sacrificing the animal. MuRF1 expression levels are also monitored to confirm that MuRF1 is being expressed by mice in which muscle atrophy is induced.

Mice in which muscle atrophy is initiated are administered either candidate compounds or compounds that inhibited muscle atrophy in myotubes in example 2. A reduction in the expression levels of FP635 or luciferase or SEAP indicates that a candidate compound inhibits muscle cell atrophy *in vivo.*

### Example 4

### Generation of reporter construct 2

The plasmid shown in Fig. 2 is modified by replacing the hluc2CP ORF with an ORF encoding the CRE recombinase and by cloning in flanking mouse genomic sequences and appropriate elements to allow gene knock-in by homologous recombination. Flanking sequences from the 3'UTR region of mouse MuRF1 gene for mediating homologous recombination are cloned in before the HCV IRES sequence and after the synthetic poly A sequence. A nucleotide sequence encoding thymidine kinase is included as a negative selection marker.

The resulting knock-in plasmid is transfected into ES cells. Cells that grow in the presence of hygromycin and gancyclovir are selected as cells in which the reporter construct is correctly targeted. Correct targeting of the reporter construct into the 3'UTR region of the MuRF1 gene is confirmed by sequencing.

### Example 5

### Animal Model for Muscle Cell Atrophy 2

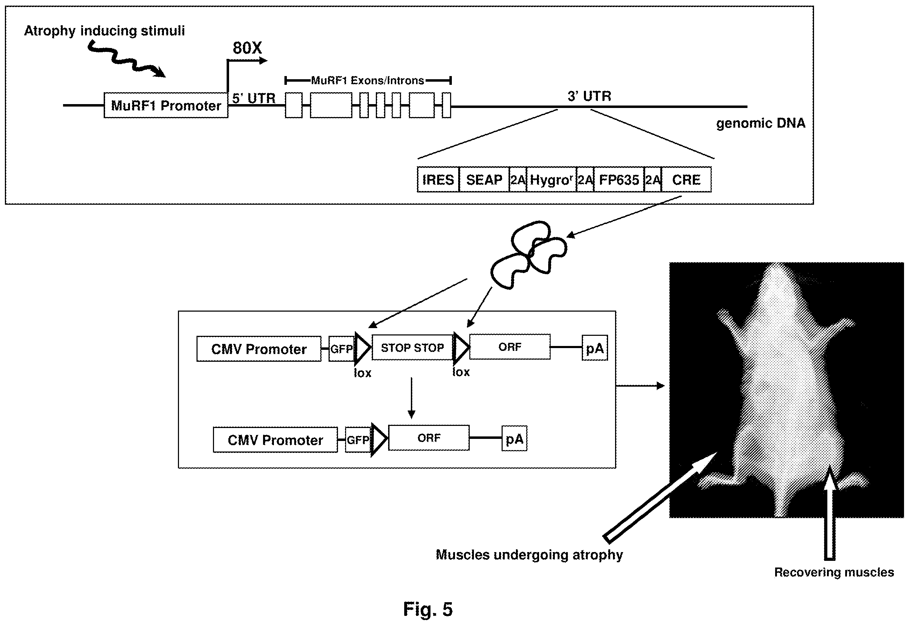

The ES cells with the correctly targeted reporter construct generated in Example 4 are used to generate transgenic mouse lines using standard procedures. The genomic region of cells in mouse lines generated from such ES cells is depicted in Fig. 5. These mouse lines are crossed with a transgenic mouse line having a construct in which the nucleic acid sequence encoding green fluorescent protein (GFP) is disrupted by the presence of stop codons that are flanked by lox sequences. The mice produced from this cross have a reporter construct, encoding SEAP, hygromycin resistance, turboFP635 and CRE, inserted into the 3'UTR of the MuRF1 gene and a copy of a construct in which the nucleic acid sequence encoding GFP is disrupted by the presence of stop codons that are flanked by lox sequences. These mice are administered dexamethasone to induce muscle atrophy. Muscles in which atrophy is initiated express CRE. CRE recombines the lox sites flanking the stop codons resulting in the excision of the stop codons and expression of GFP. Since GFP is expressed from a constitutive promoter, muscles in which atrophy was initiated are permanently marked by GFP expression. Thus, the progression of muscle atrophy after induction of atrophy may be monitored by *in vivo* imaging of either the FP635 fluorescent protein or of GFP. As muscle atrophy progresses there is an increase in the expression levels of FP635 and SEAP. Mice are administered either candidate compounds or compounds that inhibited muscle cell atrophy in myotubes in example 2. A reduction in the expression levels of FP635 or SEAP indicates that a candidate compound inhibits muscle cell atrophy *in vivo.* The degree of muscle wasting is measured by the area of the muscle tissue expressing GFP. The area decreases as atrophy progresses. However, if a compound inhibits muscle cell atrophy, this area remains the same or may even increase in size. Thus, GFP serves as a way of measuring recovery of the muscles after atrophy has been inhibited. A schematic of this system is shown in Fig. 5. Fig. 5 shows the reporter construct inserted into the 3'UTR region of mouse MuRF1 gene. Activation of the MuRF1 promoter by a muscle atrophy inducing stimuli results in expression of SEAP, FP635 and CRE. CRE recombines the lox sites flanking the stop codons that disrupt the ORF for GFP, leading to expression of GFP. Thus, the muscles in which atrophy is initiated are detected by expression of FP635 and GFP. When the MuRF1 promoter is inactivated, for example, due to inhibition of muscle atrophy, FP635 is not detectable but GFP continues to be expressed (shown in green), while muscle cells which in which the MuRF1 promoter is active would express both FP635 and GFP (shown in red).

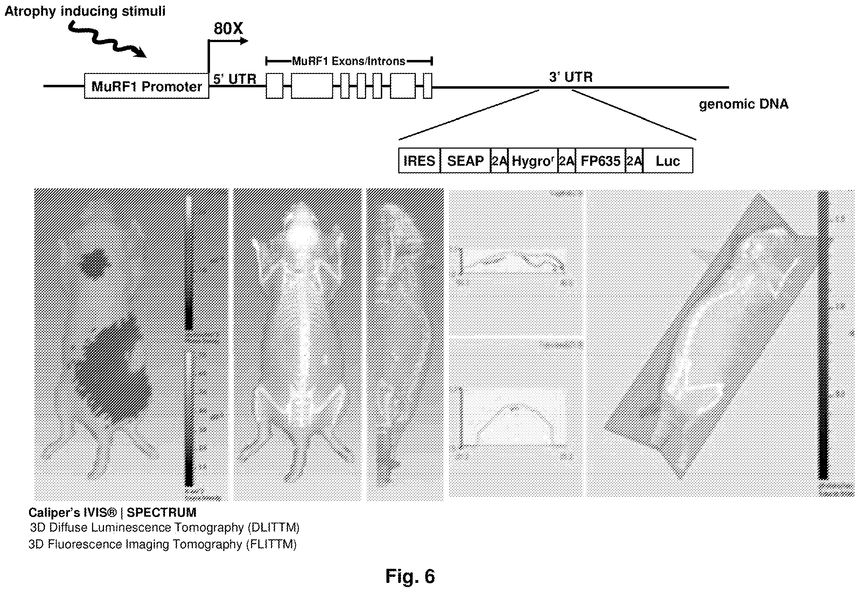

The direct volume of GFP marked muscles may be measured using whole animal imaging techniques such as 3D tomography as depicted in Fig. 6. Fig. 6 shows that 3D tomography is may be used to detect luminescence or fluorescence.

## Claims

1. A non-human animal model for muscle cell atrophy comprising:
muscle cells comprising a nuclear genome comprising a biologically active atrogen gene encoding a functional protein, whose expression is induced in muscle cells exposed to an atrophy-inducing stimulus, in operable linkage with a reporter construct present in the 3' untranslated region (UTR) or the 5' UTR of said atrogen gene, said reporter comprising:
i. a first coding sequence for a fluorescent or luminescent detectable protein; and
ii. a second coding sequence for a secreted reporter enzyme;
wherein said secreted reporter enzyme and said detectable protein are induced by muscle cell atrophy and the production of said secreted reporter of said non-human animal can be non-invasively monitored in bodily fluids and said detectable protein of said non-human animal can be monitored by *in vivo* imaging.

2. The non-human animal model of claim 1, wherein induction of said atrogen gene by muscle cell atrophy results in secretion of said secreted reporter enzyme into the bloodstream of said animal.

3. The non-human animal model of claims 1 or 2, wherein said biologically active atrogen gene is endogenous to said cell.

4. The non-human animal model of claims 1 or 2, wherein said biologically active atrogen gene is not endogenous to said cell.

5. The non-human animal model of claim 4, wherein said cell further comprises an endogenous atrogen gene, and said biological active atrogen gene is comprised in a recombinant construct that is present at a different locus to the endogenous atrogen gene.

6. The non-human animal model of any of claims 1-5, wherein said reporter construct encodes a translational shunt sequence that is present between said first coding sequence and said second coding sequence.

7. The non-human animal model of claim 6, wherein said translational shunt sequence is the sequence of the FMDV 2A translational shunt.

8. The non-human animal model of any of claims 1-7, wherein said reporter construct encodes a translational shunt sequence that bridges the final exon of said atrogen gene with said first coding sequence.

9. The non-human animal model of any of claims 1-7, wherein said reporter construct comprises an internal ribosome entry site (IRES) and is present in the 3' UTR of said atrogen gene.

10. The non-human animal model of claim 9, wherein said report construct encodes a translational shunt sequence that is present between said first coding sequence and said second coding sequence.

11. A method of screening for candidate agents that modulate muscle cell atrophy comprising:
a) contacting a non-human animal model of claim 1 with a candidate agent under conditions that induce muscle cell atrophy; and
b) determining if said candidate agent alters production of said secreted reporter enzyme and said detectable protein by said non-human animal modela.

12. The method of claim 11, wherein said conditions that induce muscle cell atrophy comprise contacting said cell with a muscle cell atrophy inducing agent.

13. The method of claim 11 further comprising measuring the activity of the secreted reporter enzyme in the blood of the animal and imaging said animal to detect expression of the detectable protein.

## Patentansprüche

1. Nichtmenschliches Tiermodell für Muskelzellenatrophie, das Folgendes umfasst:
Muskelzellen, umfassend ein Kerngenom, das ein biologisch aktives Atrogen-Gen umfasst, das für ein funktionelles Protein kodiert, dessen Expression in Muskelzellen ausgelöst wird, die einem Atrophie-induzierenden Stimulus ausgesetzt sind, operabel verbunden mit einem Reporterkonstrukt, das in der untranslatierten 3'-Region (UTR) oder der 5'-UTR des Atrogen-Gens vorhanden ist, wobei der Reporter Folgendes umfasst:
i. eine erste Kodiersequenz für ein fluoreszierend oder lumineszierend detektierbares Protein; und
ii. eine zweite Kodiersequenz für ein sekretiertes Reporterenzym;
worin das sekretierte Reporterenzym und das detektierbare Protein durch Muskelzellenatrophie induziert werden und die Produktion des sekretierten Reporters des nichtmenschlichen Tiers nichtinvasiv in Körperflüssigkeiten überwacht werden kann und das detektierbare Protein des nichtmenschlichen Tiers durch In-vivo-Bild-gebung überwacht werden kann.

2. Nichtmenschliches Tiermodell nach Anspruch 1, wobei die Induktion des Atrogen-Gens durch Muskelzellenatrophie zur Sekretion des Reporterenzyms in den Blutkreislauf des Tiers führt.

3. Nichtmenschliches Tiermodell nach Anspruch 1 oder 2, wobei das biologisch aktive Atrogen-Gen endogen zu der Zelle ist.

4. Nichtmenschliches Tiermodell nach Anspruch 1 oder 2, wobei das biologisch aktive Atrogen-Gen nicht endogen zu der Zelle ist.

5. Nichtmenschliches Tiermodell nach Anspruch 4, wobei die Zelle weiters ein endogenes Atrogen-Gen umfasst und das biologisch aktive Atrogen-Gen in einem rekombinanten Konstrukt umfasst ist, das an einem anderen Locus als das endogene Atrogen-Gen vorhanden ist.

6. Nichtmenschliches Tiermodell nach einem der Ansprüche 1 bis 5, wobei das Reporterkonstrukt für eine Translations-Shunt-Sequenz kodiert, die zwischen der ersten Kodiersequenz und der zweiten Kodiersequenz vorhanden ist.

7. Nichtmenschliches Tiermodell nach Anspruch 6, wobei die Translations-Shunt-Sequenz die Sequenz des FMDV-2A-Translationsshunts ist.

8. Nichtmenschliches Tiermodell nach einem der Ansprüche 1-7, wobei das Reporterkonstrukt für eine Translations-Shunt-Sequenz kodiert, die das letzte Exon des Atrogen-Gens mit der ersten Kodiersequenz überbrückt.

9. Nichtmenschliches Tiermodell nach einem der Ansprüche 1-7, wobei das Reporterkonstrukt eine interne Ribosomeneintrittsstelle (IRES) umfasst und in der 3'-UTR des Atrogen-Gens vorhanden ist.

10. Nichtmenschliches Tiermodell nach Anspruch 9, wobei das Reporterkonstrukt für eine Translations-Shunt-Sequenz kodiert, die zwischen der ersten Kodiersequenz und der zweiten Kodiersequenz vorhanden ist.

11. Verfahren zum Screenen auf Kandidatenmittel, die Muskelzellenatrophie modulieren, das Folgendes umfasst:
a) Kontaktieren eines nichtmenschlichen Tiermodells nach Anspruch 1 mit einem Kandidatenmittel unter Bedingungen, die eine Muskelzellenatrophie auslösen; und
b) Bestimmen, ob das Kandidatenmittel die Produktion des sekretierten Reporterenyzms und des detektierbaren Proteins durch das nichtmenschliche Tiermodell verändert.

12. Verfahren nach Anspruch 11, wobei die Bedingungen, die Muskelzellenatrophie auslösen, das Kontaktieren der Zelle mit einem eine Muskelzellenatrophie auslösenden Mittel umfassen.

13. Verfahren nach Anspruch 11, das weiters das Messen der Aktivität des sekretierten Reporterenzyms in dem Blut des Tieres und Unterziehen einer Bildgebung des Tiers zur Detektion der Expression des detektierbaren Proteins umfasst.

## Revendications

1. Modèle animal non-humain pour une atrophie de cellule musculaire, comprenant :
des cellules musculaires comprenant un génome nucléaire comprenant un gène atrogène biologiquement actif codant pour une protéine fonctionnelle, dont l'expression est induite dans des cellules musculaires exposées à un stimulus inducteur d'atrophie, en liaison opérationnelle avec une construction de rapporteur présente dans la région non traduite 3' (UTR) ou l'UTR 5 'dudit gène atrogène, ledit rapporteur comprenant :
i. une première séquence de codage pour une protéine détectable fluorescente ou luminescente ; et
ii. une seconde séquence de codage pour une enzyme rapporteur sécrétée ;
dans lequel ladite enzyme rapporteur sécrétée et ladite protéine détectable sont induites par une atrophie de cellule musculaire, et la production dudit rapporteur sécrété dudit animal non humain peut être surveillée de manière non invasive dans des fluides corporels, et ladite protéine détectable dudit animal non humain peut être surveillée par imagerie *in vivo.*

2. Modèle animal non humain selon la revendication 1, dans lequel l'induction dudit gène atrogène par atrophie de cellule musculaire entraîne une sécrétion de ladite enzyme rapporteur sécrétée dans la circulation sanguine dudit animal.

3. Modèle animal non humain selon la revendication 1 ou 2, dans lequel ledit gène atrogène biologiquement actif est endogène à ladite cellule.

4. Modèle animal non humain selon la revendication 1 ou 2, dans lequel ledit gène atrogène biologiquement actif est non endogène à ladite cellule.

5. Modèle animal non humain selon la revendication 4, dans lequel ladite cellule comprend en outre un gène atrogène endogène, et ledit gène atrogène biologiquement actif est compris dans une construction recombinante qui est présente au niveau d'un locus différent du gène atrogène endogène.

6. Modèle animal non humain selon l'une quelconque des revendications 1 à 5, dans lequel ladite construction de rapporteur code pour une séquence de dérivation translationnelle qui est présente entre ladite première séquence de codage et ladite seconde séquence de codage.

7. Modèle animal non humain selon la revendication 6, dans lequel ladite séquence de dérivation translationnelle est la séquence de la dérivation translationnelle FMDV 2A.

8. Modèle animal non humain selon l'une quelconque des revendications 1 à 7, dans lequel ladite construction de rapporteur code pour une séquence de dérivation translationnelle qui relie l'exon final dudit gène atrogène et ladite première séquence de codage.

9. Modèle animal non humain selon l'une quelconque des revendications 1 à 7, dans lequel ladite construction de rapporteur comprend un site d'entrée de ribosome interne (IRES), et est présente dans l'UTR 3' dudit gène atrogène.

10. Modèle animal non humain selon la revendication 9, dans lequel ladite construction de rapporteur code pour une séquence de dérivation translationnelle qui est présente entre ladite première séquence de codage et ladite seconde séquence codante.

11. Procédé de criblage pour des agents candidats qui modulent l'atrophie de cellule musculaire comprenant les étapes consistant à :
a. mettre en contact un modèle animal non humain selon la revendication 1 avec un agent candidat dans des conditions qui induisent une atrophie de cellule musculaire ; et
b. déterminer si ledit agent candidat altère la production de ladite enzyme rapporteur sécrétée et ladite protéine détectable par ledit modèle animal non humain.

12. Procédé selon la revendication 11, dans lequel lesdites conditions qui induisent une atrophie de cellule musculaire comprennent la mise en contact de ladite cellule avec un agent inducteur d'atrophie de cellule musculaire.

13. Procédé selon la revendication 11, comprenant en outre la mesure de l'activité de l'enzyme rapporteur sécrétée dans le sang de l'animal et l'imagerie dudit animal pour détecter l'expression de la protéine détectable.
